(19) Europäisches Patentamt
European Patent Office
Office européen des brevets

(11) Publication number: **0 052 962 B1**

(12) **EUROPEAN PATENT SPECIFICATION**

(45) Date of publication of patent specification: **09.05.84**

(21) Application number: **81305204.0**

(22) Date of filing: **02.11.81**

(51) Int. Cl.[3]: **A 61 K 31/43,**
A 61 K 31/545, A 61 K 9/00
//(A61K31/43, 31/42),
(A61K31/545, 31/42)

(54) **Pharmaceutical compositions containing two beta-lactam derivatives.**

(30) Priority: **20.11.80 GB 8037235**

(43) Date of publication of application:
**02.06.82 Bulletin 82/22**

(45) Publication of the grant of the patent:
**09.05.84 Bulletin 84/19**

(84) Designated Contracting States:
**AT BE CH DE FR GB IT LI LU NL SE**

(56) References cited:
**EP - A - 0 008 525**
**GB - A - 1 463 563**

**CHEMICAL ABSTRACTS, vol. 73, no. 5, August 3, 1970 page 244, abstract 28921g Columbus, Ohio, US**

**CHEMICAL ABSTRACTS, vol. 75, no. 8, August 23, 1971 page 245, abstract 52822n Columbus, Ohio, US**

(73) Proprietor: **BEECHAM GROUP PLC**
**Beecham House Great West Road**
**Brentford Middlesex (GB)**

(72) Inventor: **Taskis, Charles Bernard**
**Hillside Durrington Hill**
**Worthing Sussex (GB)**
Inventor: **Ashwin, Jean Gladys**
**Larkrise 35 Long Meadow**
**Worthing Sussex (GB)**

(74) Representative: **Hesketh, Alan, Dr. et al,**
**European Patent Attorney Beecham Pharmaceuticals Great Burgh Yew Tree Bottom Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 052 962 B1**

Courier Press, Leamington Spa, England.

**Description**

This invention relates to pharmaceutical compositions and, in particular, to anti-bacterial pharmaceutical compositions which may be administered by injection.

British Patent Specification No. 1,508,977 describes *inter alia* clavulanic acid of formula (I):

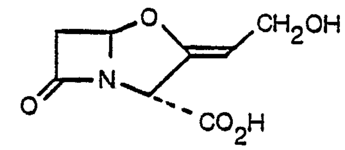

(I)

and salts thereof, and the use of such compounds as synergists for penicillins and cephalosporins.

Synergistic formulations for injection comprising clavulanic acid or salts thereof are described in the aforementioned British Patent and in European Patent Application Publication No. 0008905. Certain of these compositions tend to lose potency fairly quickly on reconsitution in the solvent for injection. It is an object of this invention to provide an injectable pharmaceutical composition having improved stability relative to the previously known compositions.

Accordingly the present invention provides an injectable pharmaceutical composition, which composition comprises: (a) a water soluble salt of clavulanic acid; (b) a water soluble salt of aplicillin, amoxycillin, epicillin, cephalexin, cephradine or cephalogycine: and (c) a vehicle of water and glycofurol.

In one aspect, the present invention provides an injectable pharmaceutical composition, which composition comprises a solution of: (a) a water soluble salt of clavulanic acid; (b) a water soluble salt of ampicillin, amoxycillin, epicillin, cephalexin, cephradrine or cephalogycine; and (c) a vehicle of water and glycofurol; and (d) optionally a local anaesthetic such as lignocaine or benzyl alcohol.

Glycofurol is the recognised term in the art for tetrahydrofurfuryl alcohol polyethylene glycol ether with an average of two ethylene glycol groups. Glycofurol may be regarded as having the formula (II):

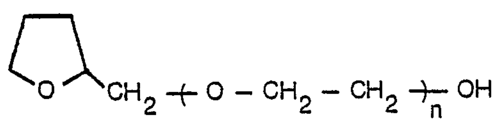

(II)

wherein n is an integer from 1 to 4, preferably 1 or 2. Suitably the glycofurol is 'Glycofurol 75' supplied by Roche (Glycofurol 75 is a trade name).

The ratio of glycofurol to water in the compositions of this invention is in the range 70:30 to 10:90, usually in the range 60:40 to 20:80, preferably in the range 50:50 to 20:80 for example approximately 50:50 or approximately 25:75 (these ratios are volume/volume). Of these 25:75 is preferred.

The ratio of anti-bacterial agents to vehicle present in the composition is normally from 2:15 to 7:15 usually 2:15 to 4:15, and generally approximates to 3:15. [These ratios are weight (taken as free acid equivalent weight)/volume].

The ratio of clavulanate salt to penicillin or cephalosporin salt present in the composition will normally be in the range 10:1 to 1:10, usually in the range 1:1 to 1:8, and will preferably be 1:2 to 1:5, for example 1:2. [These ratios are weight/weight ratios based on free acid equivalent weights].

Suitably the clavulanate salt will be the sodium or potassium salt, preferably the potassium salt.

Suitably the ampicillin, amoxycillin, epicillin, cephradine cephalexin or cephologlycine salt is the sodium or potassium salt, preferably the sodium salt.

It has been found that this invention is particularly pertinent to formulations wherein the penicillin is amoxycillin.

The formulations of this invention are particularly applicable for cephalexin, cephalogycine and epicillin which show a tendency to dimerise in aqueous solution at pH values greater than 7.0.

Thus one preferred embodiment of this invention is an injectable pharmaceutical composition comprising a solution of sodium or potassium clavulanate and sodium or potassium amoxycillin in a vehicle of water and glycolfurol wherein the ratio of gylcofuranol:water is from 70:30 to 10:90 v/v.

In these preferred embodiments the relative proportions of the ingredients are suitably as hereinbefore.

Most preferably in these embodiments the active ingredients are potassium clavulanate and sodium amoxycillin.

In another aspect, the invention also provides a twin pack in which the first part of the pack contains the active ingredients and the second part of the pack contains the vehicle.

The invention also provides a process for preparing the composition of the invention, which process comprises bringing into association the active ingredients and the vehicle.

The exact nature of this process, of course, varies with the nature of the compositions to be prepared.

When the composition is in solution form, then the process comprises dissolving the active ingredients (eigher simultaneously or consecutively) in the vehicle.

When the composition is in the twin pack form, then the process comprises filling the appropriate ingredients into separate parts of the pack.

The twin pack of this invention will generally comprise a vial containing the mixed active ingredients in the form of a dry powder and/or a freeze dried plug and a second vial or ampule containing the mixed solvents. In order to form the injectable solution the liquid from the second vial will be transferred to the first vial.

The vial containing the active ingredients is conveniently filled by freeze drying a solution of the clavulanate salt *in situ* and thereafter adding to the resulting plug the penicillin or cephalosporin salt in powder form. These dry components may then be sealed into a vial. Alternatively the vial may be filled in a convenient conventional manner with separately prepared dry salts in powder form.

The injectable compositions of this invention may be used in the manner described in U.K. Patent No. 1,508,977.

In yet a further aspect, the invention provides a method of improving the stability of an aqueous injectable solution of a water soluble salt of clavulanic acid and a water soluble salt of ampicillin, amoxycillin, epicillin, cephradine, cephalexin or cephalogycine which method comprises incorporating in the solution glycofurol as hereinbefore described.

The following Examples illustrate the invention.

Example 1

| | |
|---|---|
| Amoxycillin (as the sodium salt) | 250 mg |
| Clavulanic Acid (as the potassium salt) | 125 mg |
| Lignocaine hydrochloride | 20 mg |
| Solvent (50% water for injection 50% glycofurol) to | 2 ml |

| | |
|---|---|
| $t_{90}$*: amoxycillin | 35 min |
| clavulanic acid | 45 min |

*$t_{90}$ is defined as the time taken for the component to degrade to 90% of its inital value.

| Example no. | Amoxycillin (mg) | Clavulanic acid (mg) | Solvent (% glycofurol/ % Water) | Total Volume (ml) | $t_{90}$ (min) Clavulanic acid | $t_{90}$ (min) Amoxycillin |
|---|---|---|---|---|---|---|
| 2 | 250 | 125 | 0/100 | 2 | 8 | 12 |
| 3 | 250 | 125 | 50/50 | 1.5 | 30 | 20 |
| 4 | 250 | 125 | 50/50 | 3.0 | 75 | 95 |
| 5 | 250 | 125 | 30/70 | 2.5 | 40 | 35 |
| 6 | 250 | 125 | 40/60 | 2.0 | 40 | 30 |
| 7 | 500 | 100 | 0/100 | 3.5 | 5 | 10 |
| 8 | 500 | 100 | 50/50 | 3.5 | 40 | 70 |
| 9 | 250 | 125 | 25/75 | 1.8 | 17 | 28 |
| 10 | 500 | 125 | 25/75 | 3.5 | 15 | 39 |

Amoxycillin was used as the Sodium Salt.
Clavulanic acid was used as the Potassium Salt.
All samples included 1% w/v lignocaine hydrochloride.

**Claims for the contracting States BE CH DE FR GB IT LI LU NL SE**

1. An injectable pharmaceutical composition, which composition comprises: (a) a water soluble salt of clavulanic acid; (b) a water soluble salt of ampicillin, amoxycillin, epicillin, cephradine, cephalexin or cephaloglycine; and (c) a vehicle of water and glycofurol wherein the ratio of glycofurol:water is from 70:30 to 10:90 v/v.

2. A pharmaceutical composition as claimed in claim 1 which comprises a local anaesthetic.

3. A pharmaceutical composition as claimed in claim 1 or claim 2 wherein the ratio of glycofurol:water is approximately 25:75 v/v.

4. A pharmaceutical composition as claimed in any of claims 1 to 3 wherein the ratio of antibacterial agents to vehicle present is from 2:15 to 715 wt/vol.

5. A composition as claimed in any of claims 1 to 4 wherein the salt of clavulanic acid is sodium or potassium.

6. A pharmaceutical composition as claimed in any of claims 1 to 5 which composition comprises a solution of sodium or potassium clavulanate and sodium or potassium amoxycillin in a vehicle of glycofurol and water.

7. A twin pack in which the first part of the pack contains a water soluble salt of clavulanic acid and a water soluble salt of ampicillin, amoxycillin, epicillin, cephradine, cephalexin or cephaloglycine, and the second part of the pack contains a vehicle of glycofurol and water wherein the ratio of glycofurol:water is from 70:30 to 10:90 v/v.

8. A method for improving the stability of an aqueous injectable solution of a water soluble salt of clavulanic acid and a water soluble salt of ampicillin, amoxycillin, epicillin, cephradine, cephalexin or cephaloglycine which method comprises the incorporation of glycofurol into the solution wherein the ratio of glycofurol:water is from 70:30 to 10:90 v/v.

9. A process for preparing a pharmaceutical composition according to any of claims 1 to 8 which process comprises bringing into association the active ingredients and the vehicle.

**Claims for the contracting State AT**

1. A process for preparing an injectable pharmaceutical composition comprising: (a) a water soluble salt of clavulanic acid; (b) a water soluble salt of ampicillin, amoxycillin, epicillin, cephradine, cephalexin or cephaloglycine; and (c) a vehicle of water and glycofurol wherein the ratio of glycofurol: water is from 70:30 to 10:90 v/v, which process comprises bringing into association the active ingredients and the vehicle.

2. A process as claimed in claim 1 wherein the pharmaceutical composition comprises a local anaesthetic.

3. A process as claimed in claim 1 or claim 2 wherein the ratio of glycofurol: water is approximately 25:75 v/v.

4. A process as claimed in any of claims 1 to 3 wherein the ratio of antibacterial agents to vehicle present is from 2:15 to 7:15 wt/vol.

5. A process as claimed in any of claims 1 to 4 wherein the salt of clavulanic acid is sodium or potassium.

6. A process as claimed in any of claims 1 to 5 which composition comprises a solution of sodium or potassium clavulanate and sodium or potassium amoxycillin in a vehicle of glycofurol and water.

7. A method for improving the stability of an aqueous injectable solution of a water soluble salt of clavulanic acid and a water soluble salt of ampicillin, amoxycillin, epicillin, cephradine, cephalexin or cephaloglycine which method comprises the incorporation of glycofurol into the solution wherein the ratio of glycofurol:water is from 70:30 to 10:90 v/v.

**Patentansprüche fur der Vertragsstaaten: BE CH LI DE FR GB IT LU NL SE**

1. Eine injizierbare pharmazeutische Zusammensetzung, welche umfaßt:

(a) ein wasserlösliches Salz der Clavulansäure;

(b) ein wasserlösliches Salz von Ampicillin, Amoxycillin, Epicillin, Cephradin, Cephalexin oder Cephaloglycin; und

(c) eine Trägersubstanz aus Wasser und Glycofurol, wobei das Verhältnis von Glycofurol zu Wasser von 70:30 bis 10:90 Vol/Vol. beträgt.

2. Eine pharmazeutische Zusammensetzung wie in Anspruch 1 beansprucht, welche ein Lokalanästhetikum enthält.

3. Eine pharmazeutische Zusammensetzung wie in Anspruch 1 beansprucht, wobei das Verhältnis von Glycofurol zu Wasser ungefähr 25:75 Vol/Vol. beträgt.

4. Eine pharmazeutische Zusammensetzung wie in irgendeinem der Ansprüche 1 bis 3 beansprucht, wobei das Verhältnis von antibakteriellen Wirkstoffen zur vorliegenden Trägersubstanz von 2:15 bis 7:15 Gewicht/Vol. beträgt.

5. Eine Zusammensetzung wie in irgendeinem der Ansprüche 1 bis 4 beansprucht, wobei das Salz der Clavulansäure das Natrium- oder Kaliumsalz ist.

6. Eine pharmazeutische Zusammensetzung wie in irgendeinem der Ansprüche 1 bis 5 beansprucht, welche Zusammensetzung eine Lösung von Natrium- oder Kaliumclavulanat und Natrium- oder Kaliumamoxycillin in einer Trägersubstanz aus Glycofurol und Wasser enthält.

7. Eine Zwillingspackung, wobei der erste Teil der Zwillingspackung ein wasserlösliches Salz der Clavulansäure und ein wasserlösliches Salz von Ampicillin, Amoxycillin, Epicillin, Cephradin, Cephalexin oder Cephaloglycin enthält und der zweite Teil der Packung eine Trägersubstanz aus Glycofurol und Wasser enthält, wobei das Verhältnis von Glycofurol zu Wasser von 70:30 bis 10:90 Vol/Vol beträgt.

8. Eine Methode zur Verbesserung der Stabilität einer wäßrigen injizierbaren Lösung eines wasserlöslichen Salzes der Clavulansäure und eines wasserlöslichen Salzes von Ampicillin, Amoxycillin, Epicillin, Cephradin, Cephalexin oder Cephaloglycin, welche Methode die Einverleibung von Glycofurol in die Lösung umfaßt, wobei das Verhältnis von Glycofurol zu Wasser von 70:30 bis 10:90 ist Vol/Vol ist.

9. Ein Verfahren zur Herstellung einer pharmazeutische Zusammensetzung gemäß irgendeinem der Ansprüche 1 bis 8, welches Verfahren das Zusammenbringen deraktiven Bestandteile mit der Trägersubstanz umfaßt.

**Patentansprüche fur den Vertragsstaat: AT**

1. Ein Verfahren zur Herstellung einer injizierbaren pharmazeutische Zusammensetzung umfassend:

(a) ein wasserlösliches Salz der Clavulansäure

(b) ein wasserlösliches Salz von Ampicillin, Amoxycillin, Epicillin, Cephradin, Cephalexin oder Cephaloglycin; und

(c) eine Trägersubstanz aus Wasser und Glycofurol, wobei das Verhältnis von Glycofurol zu Wasser von 70:30 bis 10:90 Vol/Vol beträgt, welches Verfahren das Zusammenbringen der aktiven Bestandteile mit der Trägersubstanz umfaßt.

2. Eine Verfahren wie in Anspruch 1 beansprucht, in welchem die pharmazeutische Zusammensetzung ein Lokalanästhetikum enthält.

3. Eine Verfahren wie in Anspruch 1 oder 2 bespurcht, in welchem das Verhältnis von Glycofurol zu Wasser ungefähr 25:75 Vol/Vol beträgt.

4. Ein Verfahren wie in irgendeinem der Ansprüche 1 bis 3 beansprucht, in welchem das Verhältnis von antibakteriellen Wirkstoffen zur vorliegenden Trägersubstanz von 2:15 bis 7:15 Gew./Vol. beträgt.

5. Eine Verfahren wie in irgendeinem der Ansprüche 1 bis 4 beansprucht, in welchem das Salz der Clavulansäure das Natrium- oder Kaliumsalz ist.

6. Eine Verfahren wie in irgendeinem der Ansprüche 1 bis 5 beansprucht, in welchem die Zusammensetzung eine Lösung von Natrium- oder Kaliumclavulanat und Natrium- oder Kaliumamoxycillin in einer Trägersubstanz aus Glycofurol und Wasser enthält.

7. Eine Methode zur Verbesserung der Stabilität einer wäßrigen injizierbaren Lösung eines wasserlöslichen Salzes der Clavulansäure und eines wasserlöslichen Salzes von Ampicillin, Amoxycillin, Epicillin, Cephradin, Cephalexin oder Cephaloglycin, welche Methode die Einverleibung von Glycofurol in die Lösung umfaßt, wobei das Verhältnis von Glycofurol zu Wasser von 70:30 bis 10:90 Vol/Vol. ist.

**Revendications pour les Etats contractants: BE CH LI DE FR GB IT LU NL SE**

1. Composition pharmaceutique injectable, cette composition renfermant: (a) un sel soluble dans l'eau d'acide clavulanique; (b) un sel soluble dans l'eau d'ampicilline, d'amoxicilline, d'épicilline, de céphradine, de céphalexine ou de céphaloglycine; et (c) un véhicle formé d'eau et de glycofurol, dans lequel le rapport entre le glycofurol et l'eau est compris entre 70:30 et 10:90 v/v.

2. Composition pharmaceutique suivant la revendication 1, caractérisée en ce qu'elle renferme un anesthésique local.

3. Composition pharmaceutique suivant la revendication 1 ou 2, caractérisée en ce que le rapport entre le glyucofurol et l'eau est d'environ 25:75 v/v.

4. Composition pharmaceutique suivalnt l'une quelconque des revendications 1 à 3, caractérisée en ce que le rapport entre les agents antibactériens et le véhicule présent est compris entre 2:15 et 7:15 p/v.

5. Composition suivant l'une quelconque des revendications 1 à 4, caractérisée en ce que le sel d'acide clavulanique est le sel de sodium ou de potassium.

6. Composition pharmaceutique suivant l'une quelconque des revendications 1 à 5, caractérisée en ce qu'elle renferme une solution de clavulanate de sodium ou de potassium et le sel de sodium ou de potassium d'amoxicilline, dans un véhicule formé de glycofurol et d'eau.

7. Emballage ou conditionnement jumelé dans lequel la première partie de l'emballage contient un sel soluble dans l'eau d'acide clavulanique et un sel soluble dans l'eau d'ampicilline, d'amoxicilline,

d'épicilline, de céphradine, de céphalexine ou de céphaloglycine, et la seconde partie de l'emballage contient un véhicule formé de glycofurol et d'eau, dans lequel le rapport entre le glycofurol et l'eau est compris entre 70:30 et 10:90 v/v.

8. Procédé pour améliorer la stabilité d'une solution aqueuse injectable d'un sel soluble dans l'eau d'acide clavulanique et d'un sel soluble dans l'eau d'ampicilline, d'amoxicilline, d'épicilline, de céphradine, de céphalexine ou de céphaloglycine, ce procédé comprenant l'incorporation de glycofurol à la solution, où le rapport entre le glycofurol et l'eau est compris entre 70:30 et 10:90 v/v.

9. Procédé pour la préparation d'une composition pharmaceutique suivant l'une quelconque des revendications 1 à 8, ce procédé consistant à associer les ingrédients actifs et le véhicule.

**Revendications pour l'Etat contractant: AT**

1. Procédé pour la préparation d'une composition pharmaceutique injectable renfermant: (a) un sel soluble dans l'eau d'acide clavulanique; (b) un sel soluble dans l'eau d'ampicilline, d'amoxicilline, d'épicilline, de céphradine, de céphalexine ou de céphaloglycine; et (c) un véhicule formé d'eau et de glycofurol, dans lequel le rapport entre le glycofurol et l'eau est compris entre 70:30 et 10:90 v/v, ce procédé consistant à associer les ingrédients actifs et le véhicule.

2. Procédé suivant la revendication 1, caractérisé en ce que la composition pharmaceutique renferme un anesthésique local.

3. Procédé suivant la revendication 1 ou 2, caractérisé en ce que le rapport entre le glycofurol et l'eau est d'environ 25:75 v/v.

4. Procédé suivant l'une quelconque des revendications 1 à 3, caractérisé en ce que le rapport entre les agents antibactériens et le véhicule présent est compris entre 2:15 et 7:15 p/v.

5. Procédé suivant l'une quelconque des revendications 1 à 4, caractérisé en ce que le sel d'acide clavulanique est le sel de sodium ou de potassium.

6. Procédé suivant l'une quelconque des revendications 1 à 5, caractérisé en ce que la composition renferme une solution de clavulanate de sodium ou de potassium et le sel de sodium ou de potassium d'amoxicilline, dans un véhicule formé de glycofurol et d'eau.

7. Procédé pour améliorer la stabilité d'une solution aqueuse injectable d'un sel soluble dans l'eau d'acide clavulanique et d'un sel soluble dans l'eau d'ampicilline, d'amoxicilline, d'épicilline, de céphradine, de céphalexine ou de céphaloglycine, ce procédé consistant à incorporer du glycofurol à la solution, où le rapport entre le glycofurol et l'eau est compris entre 70:30 et 10:90 v/v.